# EUROPEAN PATENT APPLICATION

(11) **EP 4 421 472 A1**
(43) Date of publication of application: **28.08.2024**
(21) Application number: 22872267.4
(22) Date of filing: 19.09.2022
(51) Int. Cl.: G01N 1/20, A01N 63/27, C12R 1/38

(54) **PSEUDOMONAS ATACAMENSIS CECT 30419 WHICH IMPROVES PLANT PRODUCTION AND STIMULATES SECONDARY METABOLISM IN PLANTS**

(30) Priority: 21.09.2021 ES 202130881
(71) Applicant: Biobab R&D, S.L., 35260 Agüimes (Las Palmas) (ES)
(72) Inventor: Gutierrez Albanchez, Enrique, 28925 Alcorcón (Madrid) (ES); Horche Trueba, Ignacio, 28925 Alcorcón (Madrid) (ES); Ramos Solano, Beatriz, 28224 Pozuelo de Alarcon (ES); Gutierrez Mañero, Francisco Javier, 28691 Villanueva de la Cañada (Madrid) (ES); Lucas Garcia, José Antonio, 28760 Tres Cantos (Madrid) (ES)
(86) International application number: PCT/ES2022/070592
(87) International publication number: WO 2023/047005

(57) **Abstract**

The present Invention relates to the bacterial strain *Pseudomonas atacamensis* BBB003 (CECT 30419), a microorganism of the Gram-negative group of bacteria genus *Pseudomonas,* which improves plant production and stimulates the secondary metabolism of phenolic compounds and the ability of strawberry and raspberry extracts to inhibit enzymes related to metabolic syndrome. This strain was isolated from the rhizosphere of Nicotiana glauca, on nutrient agar (PCA) and characterised morphologically, biochemically and generically by 16S gene sequencing. It can be used to increase production due to its ability to increase the photosynthetic process by mobilising nutrients and to improve plant adaptation to stress It also improves their metabolic performance by increasing the phenolic compound content in plant species of pharmacological and nutritional interest, with it being suitable for use in obtaining a larger amount of active ingredients and/or novel foods with a standardised phenol content, such as berries with a higher anthocyanin content, as well as a fruit colour enhancer.

## Description

The present invention relates to a strain of *Pseudomonas atacamensis* (BBB003, internal laboratory code) for use in the treatment of plants with the aim of improving production by mobilizing nutrients and their adaptation to stress, and inducing the synthesis of phenolic compounds of secondary metabolism with pharmacological and nutritional interest, in addition to improving the capacity of strawberry and raspberry fruit extracts to inhibit certain enzymes related to metabolic syndrome.

This strain has been deposited for patent purposes in the Spanish Type Culture Collection (CECT), dated July 29, 2021, where it has been assigned the deposit number 30419. The CECT is based in the research building of the University of Valencia, located on the Burjassot campus (DP 46100 - Valencia, Spain).

### TECHNICAL FIELD. -

The invention falls within the fields of agronomy, biotechnology, pharmacology and novel foods. This bacterial strain can serve as a basis for the preparation of different types of products aimed at increasing yield and activating the secondary metabolism of plants, thus improving adaptation to stress. These products will improve the yield and the content of bioactives of phenolic nature that can constitute active ingredients of various medicines, improve the quality of certain foods, their colouring, and improve the ability of plants to adapt to stress.

### STATE OF THE ART.-

The relevance of strains of different *Pseudomonas* species in agriculture has been widely demonstrated, especially for their ability to stimulate growth or protect plants as biocontrol agents (Sankari-Meena et al., 2019 Agriculture Application of Pseudomonas: A View on the Relative Antagonistic Potential Against Pests and Diseases). The genus *Pseudomonas* is common among soil bacteria, and they can be opportunistic pathogens in animals and plant pathogens. According to the taxonomy of the Bergeys Manual, March 2001 edition, this bacterium belongs to the Domain Bacteria, *Phylum Proteobacteria*, Class Gamma *Proteobacteria*, Order *Pseudomonadales*, Family *Pseudomonaceae.* The genus Pseudomonas is very common in the soil system, and has been repeatedly described as a protective bacterium against various plant diseases. Some bacteria of this group produce fluorescent yellow-green pigments that are easily soluble in water. Among other functions, these pigments act as siderophores (molecules capable of capturing iron from the medium for the micro-organism's metabolism). The great metabolic versatility of the genus is due to a large number of plasmids containing inducible operons for the synthesis of specific enzymes to catabolise compounds present in the medium. The presence of Pseudomonas sp. in the rhizosphere of different plants beneficially affects their physiology indicating that its selection by the plant at the rhizosphere level is very likely. The effects of this strain on the physiology of different plants indicate that the plant selects them for its benefit.

Moreover, the development of new genomic analysis technologies has made it possible to discern new species within this genus (Gomila et al., 2015 Front. Microbiol. | https://doi.org/10.3389/fmicb.2015.00214), whose beneficial effects for agriculture have not been defined. Since Migula described the genus *Pseudomonas* in 1984, up to 255 species (including synonyms, http://www.bacterio.net/pseudomonas.html) have been described. In 2010, Mulet et al. defined two groups within the genus (*Pseudomonas aeruginosa* and *Pseudomonas fluorescens*) on the basis of phylogenetic analysis of 16S rRNA gene sequences and three housekeeping genes (gyrB, rpoB and rpoD) comparing 107 type strains. In this regard, the species *Pseudomonas atacamensis* has been recently described (Poblete-Morales et al., https://doi.org/10.1007/s10482-020-01427-0) and, however, there is no evidence showing beneficial plant growth promoting effects of any strain of this species.

Several retrospective patent searches carried out worldwide confirm this conclusion drawn from the scientific literature: the non-existence of patent documents relating to the bacterial species *Pseudomonas atacamensis* with nutrient mobilising and stress adaptation capacity, which makes it useful for improving plant growth and crop production, and of *Pseudomonas atacamensis* as an inducer of the secondary metabolism of phenolic compounds with pharmacological and nutritional interest.

### INVENTION. -

The object of the invention described herein and which, in view of the state of the prior art, is understood to meet the conditions of novelty and inventive activity necessary to be worthy of the patent right, is the isolation and characterization of the bacterial strain *Pseudomonas atacamensis* BBB003, with CECT deposit number 30419, which is a microorganism from the group of Gram-negative bacteria, genus Pseudomonas, with a demonstrated ability to mobilize nutrients that improve the photosynthetic process and metabolic performance, improving adaptation to stress conditions, functionalities that result in an increase in plant growth and production, and induce the synthesis of phenolic compounds of secondary metabolism in plant species that, due to the nature of such compounds, are of pharmacological and/or nutritional interest, in addition to have application in improving the color of certain fruits.

The physiological characteristics and genetic analysis of this strain allow it to be unequivocally identified, differentiating it from other species of the *Pseudomonas* genus.

Once isolated and characterized, various tests were carried out to reveal biochemical activities indicative of its potential capacity to mobilize nutrients and promote plant growth. These were auxin production, degradation of 1-aminocyclopropane-1-carboxylate, nitrogen mobilization, solubilization of phosphate, calcium, and production of siderophores and chitinases.

To date, experiments have been carried out consisting of the direct inoculation of the strain in a model plant (*Arabidopsis thaliana*) where it has induced a decrease in oxidative stress by decreasing the activity of superoxide dismutase (SOD), ascorbate peroxidase (APX) and catalase (CAT), enzymes involved in the elimination of free radicals (ROS), indicating better adaptation to stress. Likewise, it has been found that the plant responds to the strain by decreasing chitinase activity. When plants treated with BBB003 are attacked by the pathogen *X.campestris*, the incidence of disease is 50% lower than in untreated plants and oxidative stress decreases, presenting lower levels of SOD, APX and CAT, this decrease being much more pronounced in plants subjected to stress.

Another experiment was carried out on *Hypericum perforatum*, *Hypericaceae* family, known as St. John's Wort and characterized by containing phenolic active ingredients with antidepressant effect. This experiment under controlled conditions revealed the ability of BBB003 to stimulate growth and increase hypericin and pseudohypericin content by 200 and 40%, respectively.

On the other hand, elicitation experiments have been carried out with *Pseudomonas atacamensis* BBB003 can also be applied to any species of plants that make up red fruits or berries, such as strawberry, raspberry, blackberry, blueberry, or bilberry, in order to increase production and its content of phenolic compounds of pharmacological and nutritional interest. In particular, the application of the referred bacterial strain has been tested in raspberry (*Rubus idaeus* var. Adelita), a species in which it advances and increases production and modifies the polyphenol profile, improving the beneficial potential on health, specifically, on the metabolism and absorption of sugars; In strawberry (*Fragaria x ananasa* var. Fortuna), the modification of polyphenols results in an increase of anthocyanins representing an improvement of the market price due to a better coloration, especially important in early productions, which improves the commercial value of the fruit.

In another field test, it has been found that *Pseudomonas atacamensis* combined with *Bacillus amyloliquefaciens* CECT9371 also increases the anthocyanin content in strawberries. In blackberry, combined with *Bacillus amyloliquefaciens* CECT9371 and Pseudomonas fluorescens CECT9015, it anticipates and increases the production and content of anthocyanins, stimulating the fixation of CO2 and increasing photosynthetic pigments.

In field tests, it has been found that *Pseudomonas atacamensis* combined with another strain of *Pseudomonas: P. putida* CECT 9011, improves the production of melon and industrial tomato, advancing ripening and increasing the number of fruits; and in another field test, it has been found that combined with two strains of Pseudomonas: *P. fluorescens* CECT 9015 and *P. putida* CECT 9011 also improve the commercial production of industrial tomatoes.

The aforementioned experiments on the use of *Pseudomonas atcamensis* BBBB003 as a production enhancer, stress adaptation enhancer and elicitor of the secondary metabolism of phenolic compounds are set out at the end of this report, within the embodiment section.

The purpose pursued, ultimately, with this invention and which constitutes the technical advantage provided by it, is to have a bacteria that improves agricultural production and stimulates the secondary metabolism of phenolic compounds in plants of pharmacological and nutritional interest, achieving a double effect: on the one hand, improving the anthocyanin content of red fruits, and therefore their commercial value, and on the other hand, obtaining the improved plant raw material to obtain extracts that improve their effect on the alpha-glucosidase and alpha-amylase enzymes, which can be used to improve symptoms or prevent metabolic syndrome. Also, due to its nutrient mobilizing effect, it improves plant production due to its ability to increase the photosynthetic process by improving carbon fixation and increasing photosynthetic pigments, as well as the mobilization of nutrients.

### GRAPHICS.-

Two figures with graphs of experimental results are included at the end of this descriptive report.

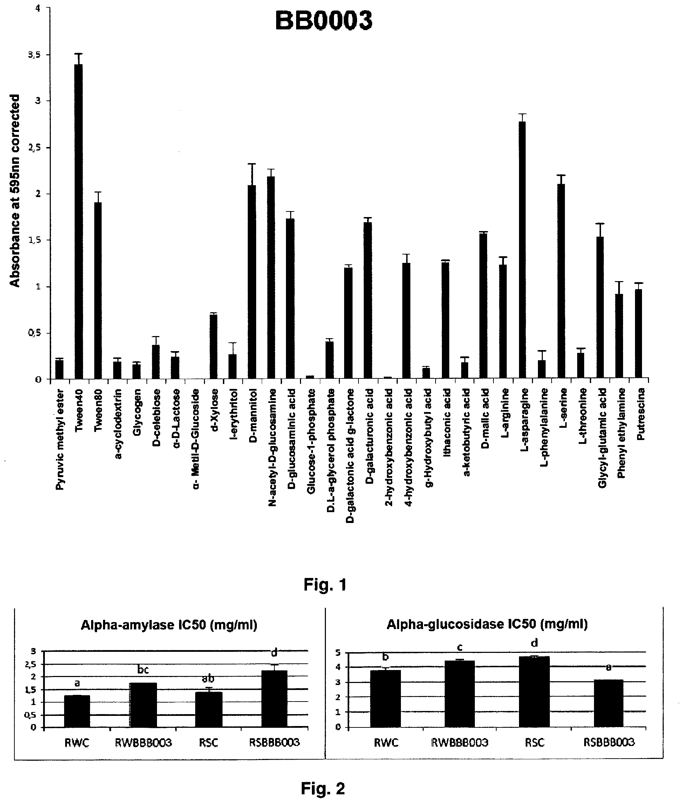

The graph in **Figure 1** shows the metabolic profile of the strain (BIOLOG-ECO), and the graph in **Figure 2** shows the inhibition capacity (IC50) of α-glucosidase and α-amylase under the influence of raspberry extracts treated with *Pseudomonas atacamensis* BBB003 and control in winter (W) and spring (S).

### METHOD OF REALISATION.-

The strain of the genus *Pseudomonas* described here was isolated by studying the rhizosphere of natural populations of *Nicotiana glauca* in a transect of the island of Las Palmas de Gran Canaria (Canary Islands). This plant species (*Nicotiana glauca*) was selected because it belongs to the Solanaceae family and has an active secondary metabolism.

Rhizosphere sampling for the isolation of the bacterial strain was carried out in natural populations of *Nicotiana glauca* in December 2017. As a result of this sampling, 450 strains were collected, among which *Pseudomonas atacamensis* (BBB003, internal laboratory code) was found. The isolation of this strain was performed on nutrient agar (PCA).

In the laboratory, this microorganism is maintained with a high survival rate in 20% glycerol in nutrient broth (Pronadisa) at -80°C or in 15% glycerol in water at -20°C and is easily recovered in the culture medium used for isolation both in solid and liquid phase at 28°C.

### Morphological, biochemical and genetic characteristics of Pseudomonas atacamensis BBB003.-

For the characterisation of the strains, different phenotypic characters were considered and are detailed in this report: (i) colony morphology, (ii) cell morphology, (iii) biolog and biochemical tests, (iv) sequencing of the ribosomal DNA gene corresponding to the 16S subunit.
(i) Colony morphology after 24 h incubation at 28°C on standard agar (PCA) is specified in Table 1:

**Table 1**

| | BBB003 |
|---|---|
| Colony size | < 1 mm∅ |
| Shape | Circular |
| Border | Smooth |
| Transparency | No |
| Consistency | Creamy |
| Colour | Pale yellow |

Growing in liquid medium (Pronadisa nutrient broth) the colour of the medium changes to yellow from the exponential phase of growth to the stationary phase of growth.
(ii) Morphological characters of *Pseudomonas atacamensis* BBB003 after 24 h incubation at 28° on standard agar (PCA) correspond to a Gram-negative bacillus.
(iii) Once isolated and characterised, with internal reference code BBB003, several tests were carried out to demonstrate the PGPR potential of this bacterium. These were auxin production (Brick et al., 1991 Appl. Environ. Microbiol., 57(2), 535; Sergeeva et al., 2007, Plant Soil, http://doi.org/10.1007/s11104-007-9314-5), degradation of 1-aminocyclopropane-1-carboxylate (Glick et al., 1995. http://doi.org/10.1139/m95-070), nitrogen mobilisation, phosphate solubilisation (De Freitas et al., 1997. Biol. Fertil. Soils, 24(4), 358-364), calcium (Tamilselvi et al., 2016. Front. plant sci, 7, 1828), and siderophore production (Alexander and Zuberer, 1991. Biol. Fertil. Soils, 12(1), 39-45) and chitinases (Frändberg and Shunürer, 1998 Can.J. Microbiol. 44: 121-127).

The metabolic profile (BIOLOG Eco, www.biolog.com/products-portfolio-overview/microbialcommunity-analysis-with-ecoplates/) of the strain, shown in **Figure 1**, indicates that it is able to degrade carboxylic acids malic acid, galacturonic acid, itaconic acid and glucosaminic acid, and the degradation of mannitol, xylose, g lactone gluconic acid and N-acetylglucosamine stand out among the sugars. As nitrogen sources, it uses putrescine, phenylethylamine, serine, arginine, asparagine and glycol-5 L-glutamic acid. And finally, as phenolic compounds, it uses hydroxybenzoic acid.

iv) Next, the taxonomic characterisation of BBB003 was carried out by means of genetic analysis of the strain for its identification, for which the following steps were followed, PCR amplification carried out using the universal *primers* 27F (AGAGTTTGATCMTGGCTCAG)/1492R (TACGGYTACCTTGTTACGACTT), and sequencing using *primers* 785F(GGATTAGATACCCTGGTA)/907R (CCGTCAATTCMTTTRAGTTT). Comparison of 16S ribosomal DNA with existing sequences in databases revealed 99.86% homology with a *Pseudomonas atacamensis* strain.

### Experiments demonstrating the ability of Pseudomonas atacamensis to enhance plant production and stimulate secondary metabolism of phenolic compounds and the ability of strawberry and raspberry extracts to inhibit enzymes related to metabolic syndrome.-

**1°.** Direct inoculation experiment of *Pseudomonas atacamensis* on a model plant (*Arabidopsis thaliana*). Experiment carried out in an experimental chamber under controlled conditions of light, humidity and temperature on a total of 42 plants and an N = 3, with a randomised block model - a cell suspension of the strain was inoculated in pre-germinated A. *thaliana* seedlings 2 and 5 weeks after germination. At 6 weeks fluorescence photosynthetic parameters (F0, Fv/Fm, PSII, NPQ) were measured, plants were harvested, and enzyme activities related to ROS scavenging (SOD, APX, CAT), and Ascorbate glutathione cycle (dehydroascorbate reductase - DHR, monodehydroascorbate reductase-MDHR, glutathione reductase-GR) were weighed and analysed. The metabolic profile of enzymes related to the control of oxidative stress (ROS scavenging) induced by the BBB003 bacterium consists of a decrease in superoxide dismutase (SOD), Ascorbate peroxidase (APX) and catalase (CAT) activity, without altering the other activities, which indicates a more relaxed state of the plant, reflecting a better adaptation to the environment.

In a similar experiment, when arabidopsis plants stimulated with BBB003 were subjected to an application of the foliar pathogen *X.campestris* 5 weeks after germination, 50% protection was recorded compared to control plants. In this case, the profile of enzymes related to the control of oxidative stress showed again a decrease in SOD, APX and CAT, being in this case a much more pronounced decrease compared to the control with pathogen. This indicates a very efficient improvement of adaptation to abiotic stress. Since the mechanisms involved in stress adaptation are always mediated by free radicals, this behaviour can be applied to any type of stress.

**2°.** Elicitation experiment on *Hypericum perforatum* plants by direct application of bacterial suspensions to the root. A bacterial suspension of strain BBB003 was inoculated into the root of *Hypericum perforatum* seedlings two weeks after germination and subsequently inoculated every two weeks for 12 weeks. Induction of the synthesis of the active principles hypericin and pseudohypericin was observed, leading to an increase in the concentration of these active principles.

**3°.** Direct inoculation experiment of *Pseudomonas atacamensis* on raspberry (*Rubus* var. Lochness) in production greenhouses. Experiment carried out under real field production conditions on a total of 400 plants and n=3 for each treatment (control and bacteria), with a randomised block experimental model. Cell suspensions of the strain were applied at root level during the 10-month production cycle, twice a month, from October to May. Total production was collected and two sampling moments were determined, in full fruiting, at the two production peaks in January and May. Bioactives (phenols and anthocyanins) were determined in fruit, nutritional (pH, °Brix, and % citric acid); and finally, measurements were made with methanolic fruit extracts on the inhibition of enzymes related to glucose regulation (α-amylase and α-glucosidase), enzymes related to metabolic syndrome. An increase in fruit production (+16%) and earlier production, i.e. stimulation of earliness, was recorded. There was i) a change in the phenol profile compared to the control at both sampling times, ii) an increase in the ability of fruit extracts to inhibit α-glucosidase. The content of anthocyanins and phenols in fruits of inoculated and control plants in the winter (W-winter) and spring (S-spring) samples is shown in Table 2; the IC50 of α-glucosidase, and α-glucosidase of both samples, treated with *Pseudomonas atacamensis* (P) and control (C) are shown in the graph in **Figure 2****.** In this graph, the abbreviation "RWC" corresponds to a sample of methanolic extracts of raspberries harvested in winter from uninoculated plants, used as control; "RWBBB003" corresponds to a sample of methanol extracts of raspberries harvested in winter from plants inoculated with the strain; "RSC" corresponds to a sample of methanol extracts of raspberries harvested in spring from uninoculated plants, used as control; "RSBBB003" corresponds to a sample of methanol extracts of raspberries harvested in spring from plants inoculated with the strain; and the units of IC50 mg/ml refer to dry weight.

The characterisation of the extracts was carried out as follows:
The total phenolic content of the extract is determined by the colorimetric method of Singleton V.L., Rossi J.A. (1965) Colorimetry of total phenolics with phosphomolybdicdicphosphotungstic acid reagent. Am J Enol Vitic 10 16,144-158, which is based on the oxidation in basic medium of the hydroxyl groups of phenols by Folin-Ciocalteu's reagent. The results are expressed as mg gallic acid/g extract. Thus, the extracts obtained following the processes described below have a minimum total phenolic content of 20 mg/g.

The total anthocyanin content was determined by the pH differential method described by Giusti and Wrolstad (2001), Anthocyanins Characterization and measurement with UV-visible spectroscopy. In: Wrolstad RE, Acree TE, An H, Decker EA, Penner MH, Reid DS, Schwartz SJ, Shoemaker CF, Sporns P, Wiley J (ed) Current Protocols in Food Analytical Chemistry. New York, pp F121-F129. doi: 10.1002/0471142913.faf0102s00, which evaluates the different absorbance of anthocyanins at different pH. Results are expressed as mg cyanidin-3-glucoside equivalents per gram of fresh extract.

The results of the characterisation of methanol extracts from raspberries harvested in winter (W) or spring (S), from plants inoculated with *Pseudomonas atacamensis* BBB003 (P) and uninoculated controls (C), according to this experiment are shown in Table 2.

**Table 2**

| Sample | Total phenols (mg g-1 dry weight of gallic acid equivalents) | Total anthocyanins (mg g-1 dry weight of cyanidin-3-glucoside equivalents) |
|---|---|---|
| RWC | 2.57± 0.039 (a) | 1.96± 0.009 (a) |
| RWP | 2.69± 0.024 (a) | 1.94± 0.003 (a) |
| RSC | 6.35± 0.028 (c) | 4.93± 0.18 (c) |
| RSP | 5.41± 0.088 (b) | 4.27± 0.185 (b) |

**4°.** Direct inoculation experiments of *Pseudomonas atacamensis* on strawberry (*Fragaria x ananasa* var. Fortuna) in production greenhouses. Experiment carried out under real field production conditions on a total of 700 plants and n=3 for each treatment (control and bacteria), with a randomised block experimental model; the experiment was carried out in the 15/16 and 16/17 seasons with similar results. Cell suspensions of the strain were applied at root level during the 6-month production cycle, twice a month, from October to May. Total production was collected and two sampling moments were determined, incipient fruiting (January) and full fruiting (April). An improvement in nutritional parameters (brix and pH), as well as in the content of phenolic compounds, especially anthocyanins, was recorded at both sampling times, as shown in Table 3.

Table 3 shows the characterisation data of strawberries harvested in January and March from plants inoculated with *Pseudomonas atacamensis* BBB003 and non-inoculated controls. Phenol units are mg gallic acid equivalents/100 g fresh weight; flavonols are mg catechin equivalents/100 g FP; anthocyanins are mg cyanidin equivalents/100 g FP. The asterisk * indicates significant differences between the control and BBB003 in the January samples according to the t-sudent statistic; the hash # indicates significant differences between the control and BBB003 in the March samples according to t-student.

**Table 3**

| | January | | March | |
|---|---|---|---|---|
| | Control | BBB0003 | Control | BBB0003 |
| pH | 3,90 ± 0,03 | 4,19 ± 0,05 | 3,41 ± 0,04 | 3,48 ± 0,05 |
| °Brix | 8,80 ± 0,02 | 9,05 ± 0,04* | 6,70 ± 0,02 | 6,80 ± 0,05 |
| % Citric Acid | 0,80 ± 0,02 | 0,74 ± 0,02 | 0,63 ± 0,05 | 0,62 ± 0,04 |
| Phenols | 119,20 ± 1,9 | 131,41 ± 2,2* | 110,25 ± 1,7 | 111,73 ± 1,2 |
| | 5,45 ± 0,8 | 6,22 ± 0,75* | 15,45 ± 0,8 | 14,42 ± 0,9 |
| Anthocyanins | 5,40 ± 0,6 | 9,73 ± 0,9* | 10,60 ± 0,9 | 13,59 ± 1,1# |

**5°.** In an experiment similar to that described in 4th, bacterial suspensions of *Pseudomonas atacamensis* BBB003, *Bacillus amyloliquefaciens* CECT9371 and a combination of these were applied. Table 4 shows the characterisation results of strawberries harvested in January and March on plants inoculated with *Pseudomonas atacamensis* BBB003 and non-inoculated controls. Different letters indicate significant differences (p<0.05) according to ANOVA and LSD. An improvement in anthocyanin content (+21%) was again recorded with BBB003, similar to that of *Bacillus amyloliquefaciens* CECT9371, with a synergistic effect confirmed by Colby's statistical test (1967, Weeds, Vol 15, no.1.:20-22) when both bacteria were applied (+64%). The characterisation of the extracts was done as indicated in 3) for phenols and anthocyanins; flavonols according to the aluminium chloride colorimetric method of Zishen et al. (1999. Food Chem 64:555-559. doi: 20 10.1096/50308-8146(98)00102-2) with modifications. Expressed as mg catechin equivalents per 100 g fresh weight.

**Table 4**

| | Phenols (mg eq gallic acid / 100 g FP) | Flavonols (mg eq catechin / 100 g FP) | Anthocyanins (mg eq cyanidin / 100 g FP) |
|---|---|---|---|
| Control | 159± 0,11 (a) | 15,9± 0,18 (a) | 7,1± 0,06 (a) |
| P-BBB003 | 180± 0,20 (b) | 16,1 ± 0,11 (a) | 9,0± 0,10 (b) |
| B-CECT9371 | 150± 0,16 (a) | 13,4± 0,20(b) | 9,5± 0,08 (b) |
| P-BBB003+B-CECT9371 | 150± 0,12 (a) | 16,0± 0,12(a) | 11,8± 0,09 (c) |

Methanolic extracts were prepared and characterised with these strawberries as described in 3° and their ability to inhibit the enzymes α-amylase and α -glucosidase was evaluated, with the results shown in Table 5. A 17% increase in the ability of BBB003-treated fruits to inhibit α -glucosidase and a 10% increase in the ability to inhibit α -amylase was found. In this case, the combination of bacteria did not have an enhancing effect on the ability to inhibit the enzymes.

**Table 5**

| | IC50 α- glucosidase (mg/ml) | IC50 α- amylase (mg/ml) |
|---|---|---|
| Control | 155 ± 0,16 (a) | 150 ± 0,11 (a) |
| *P.atacamensis* BBB003 | 120 ± 0,15 (b) | 130 ± 0,12 (b) |
| *B.amyloliquefaciens*CECT9371 | 125 ± 0,18 (b) | 129 ± 0,14 (b) |
| P.atacamensisBBB003 + B.amyloliquefaciens CECT9371 | 159 ± 0,15 (a) | 121 ± 0,16 (b) |

This table shows IC50 dry weight values for α -glucosidase and α -amylase under the effects of strawberry extracts from plants treated with *Pseudomonas atacamensis* BBB003, *Bacillus amyloliquefaciens* CECT9371 and both bacteria. Different letters indicate significant differences (p<0.05) according to ANOVA and LSD.

**6°.** Experiments on the application of formulations containing *Pseudomonas atacamensis* in combination with other strains (*Bacillus amyloliquefaciens* CECT 9371 and *Pseudomonas fluorescens* CECT 9015) on blackberry (*Rubus* sp. Loch Ness) in production greenhouses. Experiment carried out under real field production conditions on a total of 120 plants and n=3 for each treatment (control and product), with a randomised block experimental model; the experiment was carried out in the 1920 season. The product was applied at root level during the 5 months of the production cycle, twice a month from November to April (10 applications). Photosynthesis (fluorescence and CO2 fixation) was measured, and an increase in photochemical quenching (□PSII) and an increase in CO2 fixation were found, which resulted in higher production. Photosynthetic pigments were analysed according to Harmut et al, 2001 (Harmut, K. Lichtenthaler and Claus Buschmann. Extraction of Photosynthetic Tissues: Chlorophylls and Carotenoids. Current Protocols in Food Analytical Chemistry F.4.2.1- F.4.2.1.) The total yield was collected. A yield improvement of 18.3% was recorded, as well as a yield advance (earliness), which is of high commercial value.

**7°.** Experiments on the application of formulations (containing other strains of *Pseudomonas,* specifically *Pseudomonas* putida CECT 9011 and protein hydrolysate as a base fertiliser) containing *Pseudomonas atacamensis* on melon (*Cucumis melo*, var. Piel de sapo Gran Riado) in the open field. Experiment carried out under real field production conditions on a total of 360 plants and n=3, each replicate consisting of 40 plants, for each treatment (control, base fertiliser and product), with a randomised block experimental model; the experiment was carried out in the 19/20 season. The product was applied at root level during the 3 months of the production cycle, once a month at a rate of 1 kg/ha (3 applications). The total production was collected, and an improvement of 36.9% was recorded, increasing the size and number of fruits with respect to the control and the base fertiliser.

**8°.** Experiments on the application of formulations (containing other strains of *Pseudomonas,* specifically *Pseudomonas putida* CECT 9011 and protein hydrolysate as a base fertiliser) containing *Pseudomonas atacamensis* on industrial tomato (*Solanum lycopersicum*, industrial tomato var. Malpica F1) in the open field. Experiment carried out under real field production conditions on a total of 360 plants and n=3, each replicate consisting of 40 plants, for each treatment (control, base fertiliser and product), with a randomised block experimental model; the experiment was carried out in the 1920 season. The product was applied at root level during the 4 months of the production cycle, once a month at a rate of 1 kg/ha (4 applications). The total yield was collected. A 16% improvement in production was recorded, with an increase in the number of fruits compared to the control and the base fertiliser.

**9°.** Experiments on the application of formulations (containing other strains of *Pseudomonas,* specifically *Pseudomonas fluorescens* CECT 9015 and *Pseudomonas putida* CECT 9011 (and protein hydrolysate as a base fertiliser) containing *Pseudomonas atacamensis* on industrial tomato (*Solanum lycopersicum*, industrial tomato var. Malpica F1) in the open field. Experiment carried out under real field production conditions on a total of 360 plants and n=3, each replicate consisting of 40 plants, for each treatment (control, base fertiliser and product), with a randomised block experimental model; the experiment was carried out in the 19/20 season. The product was applied at root level during the 4 months of the production cycle, once a month at a rate of 1 kg/ha (4 applications). The total production was collected, and an improvement of 14% was recorded, increasing the number of fruits with respect to the control and the base fertiliser.

**10°.** Experiments on the application of formulations (containing other strains of *Pseudomonas,* specifically *Pseudomonas fluorescens* CECT 9015 and protein hydrolysate as a base fertiliser) containing *Pseudomonas atacamensis* on mandarin trees, var. Orogrande. The trial was carried out on 310 trees, and the product was applied in half at a dose of 250 g/ha, once a month from April until harvest in November (8 applications). An improvement in the brix degrees of the mandarins of 10.76% with respect to the control was found, representing a commercial improvement of the fruit.

**11°.** Experiments on direct application of *Pseudomonas atacamensis* on cherry tomatoes. The trial was carried out on 140 plants, in pots with peat/sand, for 4 months. The experimental design included the following variables: place of application (root/leaves), frequency (1 or 2 times a month), and bacterial density (107, 108 cfu/ml); each treatment had 3 replicates of 5 plants each, in addition to the untreated control. Tomatoes were harvested from the first bunch to the 7th bunch, weighed and brix were determined. An improvement in brix degrees was found with respect to the control, with the most effective treatment for increasing brix degrees being 108 via the root, once a month, although there was also an improvement with respect to the control 10e8 via the foliar, once a month. With regard to the increase in production, the application via the root achieves a 35% increase in the weight of the tomatoes.

### INDUSTRIAL APPLICATION. -

The above-mentioned properties of *Pseudomonas atacamensis* BBB003 can be used to increase plant production due to its capacity to increase the photosynthetic process by improving carbon fixation and increasing photosynthetic pigments and the mobilisation of nutrients. It also improves metabolic performance by increasing the content of phenolic compounds in plant species of pharmacological and food interest, whose application would be to obtain a greater quantity of active principles and/or new foods with a standardised phenolic content, such as fruits of the forest with a higher anthocyanin content, also having application as an improver of plant adaptation to stress

## Claims

1. **Bacterial strain of the species *Pseudomonas atacamensis* with deposit number CECT 30419,** microorganism of the Gram -negative group of bacteria, genus Pseudomonas, which improves plant production and stimulates the secondary metabolism of plants, **characterized by** its capacity to mobilize nutrients that stimulate the photosynthetic process of plants, to stimulate the adaptive metabolism to stress, by modulating the oxidative response through induction of the metabolism involved in the elimination of free radicals (FR) produced in the stress situation, to stimulate the secondary metabolism of phenolic compounds, acting directly in plants of pharmacological and nutritional interest, and to improve the properties of raspberry and strawberry fruit extracts as inhibitors of enzymes related to metabolic syndrome, specifically, α-amylase and α-glucosidase, with a hypoglycemic effect.

2. Bacterial strain of the species *Pseudomonas Atacamensis* with CECT deposit number 30419, according to claim 1, **characterized by** its ability to modify the polyphenol profile in red fruits, such as strawberries and raspberries, improving their pharmacological, nutritional and coloring properties.

3. Use of the bacterial strain of the species *Pseudomonas Atacamensis* with deposit number CECT 30419, according to claim 1, for its application in any species of plants to improve plant growth and production.

4. Use of the bacterial strain of the species *Pseudomonas Atacamensis* with deposit number CECT 30419, according to claim 1, for application in any type of agricultural or forestry crop, in order to increase the production of phenolic compounds of secondary metabolism of pharmacological and nutritional interest.

5. Use of the bacterial strain of the species *Pseudomonas Atacamensis* with deposit number CECT 30419, according to claim 4, for its application in *Hypericum perforatum* (Hipericaceae) in order to increase the content of phenolic compounds of pharmacological interest: hypericin and pseudohypericin.

6. Use of the bacterial strain of the species *Pseudomonas Atacamensis* with deposit number CECT 30419, according to claim 2, for application in any species of so-called red fruits or berries, such as strawberries, raspberries or blackberries, in order to increase the content of phenolic compounds of secondary metabolism.

7. Use of the bacterial strain of the species *Pseudomonas Atacamensis* with deposit number CECT 30419, according to claim 1, for application in raspberry and strawberry fruits, in order to obtain extracts enriched in phenolic compounds with improved capacity to inhibit enzymes involved in the metabolism of sugars in humans, related to metabolic syndrome, specifically α-amylase and α-glucosidase.

8. Use of the bacterial strain of the species *Pseudomonas Atacamensis* with deposit number CECT 30419, according to claims 3 and 7, by forming part of any bacterial preparation, either individually or in combination with other organisms, and by any available means that puts the bacteria in contact with the seed, the root or aerial system of the plants.

9. Use of the bacterial strain of the species *Pseudomonas Atacamensis* with deposit number CECT 30419, according to claim 8, in combination with *Pseudomonas fluorescens* CECT 9015 and/or *Pseudomonas putida* CECT 9011 and/or *Bacillus amyloliquefaciens* CECT 9371, for application on any herbaceous or woody species, to improve plant growth and production.

10. Use of the bacterial strain of the species *Pseudomonas Atacamensis* with deposit number CECT 30419, according to claim 9, in combination with *Pseudomonas fluorescens* CECT 9015 and/or *Pseudomonas putida* CECT 9011 and/or *Bacillus amyloliquefaciens* CECT 9371, for application on blackberries, raspberries, tomatoes and melons, in order to increase production.
